# EUROPEAN PATENT SPECIFICATION

(11) **EP 0 672 057 B1**
(45) Date of publication and mention of the grant of the patent: **20.09.2000**
(21) Application number: 93915814.3
(22) Date of filing: 06.07.1993
(51) Int. Cl.: C07K 14/635, C12N 15/62, C12P 21/02, C12N 1/20, A61K 38/29

(54) **ANALOGS OF PTH**
ANALOGE DES PARATHORMONS
ANALOGUES DE PARATHORMONE

(30) Priority: 15.07.1992 GB 9215009; 18.12.1992 GB 9226415; 23.12.1992 GB 9226861; 23.12.1992 GB 9226859; 28.01.1993 GB 9301691; 28.01.1993 GB 9301692; 14.04.1993 GB 9307673; 19.04.1993 GB 9308033
(43) Date of publication of application: 20.09.1995
(73) Proprietor: Novartis AG, 4058 Basel (CH)
(72) Inventor: BAUER, Wilfried, CH-4431 Lampenberg (CH); BRECKENRIDGE, Robin, CH-4132 Muttenz (CH); CARDINAUX, François, CH-4206 Seewen (CH); GOMBERT, Frank, D-7859 Huttingen (DE); GRAM, Hermann, D-7858 Weil-Haltingen (DE); RAMAGE, Paul, CH-4153 Reinach (CH); SCHNEIDER, Helmut, San Francisco ,CA 94109 (US); WAELCHLI, Rudolf, CH-4053 Basle (CH); ALBERT, Rainer, CH-4052 Basle (CH); LEWIS, Ian, CH-4125 Riehen (CH)
(86) International application number: EP9301749
(87) International publication number: WO9402510

(56) References cited:
- EP-A- 0 477 885
- WO-A-90/10067
- WO-A-92/11286
- JOURNAL OF BIOLOGICAL CHEMISTRY, vol. 286, no. 3, 25 January 1991, MD US, pages 1997-2004, XP002034021 F.E.COHEN E.A: "Analogues of PTH modified at positions 3 and 6"
- ENDOCRINE RESEARCH COMM., vol. 2, no. 8, 1975, NEW YORK, pages 561-570, XP002034022 G.W.TREGEAR ET AL: "SYNTHETIC ANALOGS OF RESIDUES 1-34 OF HPTH;INFLUENCE OF NO.1 ON BIOLOGICAL POTENCY IN VITRO"
- BIOCHEMISTRY, vol. 31, no. 16, 28 April 1992, pages 4026-4033, XP000266280 ELIAHU ROUBINI ET AL: "SYNTHESIS OF FULLY ACTIVE BIOTINYLATED ANALOGUES OF PARATHYROID HORMONE AND PARATHYROID HORMONE-RELATED PROTEIN AS TOOLS FOR THE CHARACTERIZATION OF PARATHYROID HORMONE RECEPTORS"
- JOURNAL OF BIOLOGICAL CHEMISTRY, vol. 263, no. 3, 25 January 1988, MD US, pages 1307-1313, XP002034023 S.A.RABBANI E.A: "Recombinant hPTH synthesized in E coli"

SEQUENCE LISTING

(1) GENERAL INFORMATION:

(i) APPLICANT:
(A) NAME: SANDOZ LTD
(B) STREET: PO BOX
(C) CITY: BASEL
(D) STATE: BS
(E) COUNTRY: SWITZERLAND
(F) POSTAL CODE (ZIP): CH 4002
(G) TELEPHONE: 061 324 1111
(H) TELEFAX: 061 322 7572
(I) TELEX: 965 050 55

(ii) TITLE OF INVENTION: Organic Compounds

(iii) NUMBER OF SEQUENCES: 12

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO:1:

(i) SEQUENCE CHARACTERISTICS:
(A) LENGTH: 717 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(v) FRAGMENT TYPE: internal

(vi) ORIGINAL SOURCE:
(A) ORGANISM: Bateriophage T4/Homo sapiens

(ix) FEATURE:
(A) NAME/KEY: CDS
(B) LOCATION: 101..559

(xi) SEQUENCE DESCRIPTION: SEQ ID NO:1:

AGATCTCGAT CCCGCGAAAT TAATACGACT CACTATAGGG AGACCACAAC GGTTTCCCTC 60

TAGAAATAAT TTTGTTTAAC TTTAAGAAGG AGATATACAT ATG TCA GAA ACT AAG 115
Met Ser Glu Thr Lys
1               5

CCT AAA TAT AAT TAC GTA AAC AAT AAA GAG CTT TTA CAA GCT ATT ATT 163
Pro Lys Tyr Asn Tyr Val Asn Asn Lys Glu Leu Leu Gln Ala Ile Ile
                10                  15                  20

GAT TGG AAA ACA GAA TTA GCA AAT AAT AAA GAC CCA AAT AAA GTA GTT 211
Asp Trp Lys Thr Glu Leu Ala Asn Asn Lys Asp Pro Asn Lys Val Val
            25                  30                  35

CGT CAG AAT GAT ACT ATC GGA TTA GCC ATT ATG CTT ATT GCA GAA GGC 259

Arg Gln Asn Asp Thr Ile Gly Leu Ala Ile Met Leu Ile Ala Glu Gly
        40                  45                  50

TTA TCT AAA CGT TTC AAC TTT TCA GGA TAC ACC CAG TCT TGG AAA CAA 307
Leu Ser Lys Arg Phe Asn Phe Ser Gly Tyr Thr Gln Ser Trp Lys Gln
    55                  60                  65

GAA ATG ATT GCA GAT GGT ATA GAA GCT TCT ATT AAG GGG CTT CAC AAT 355
Glu Met Ile Ala Asp Gly Ile Gl[?] Ala Ser Ile Lys Gly Leu His Asn
70 75 80 85

TTT GAT GAA ACG AAA TAT AAA AAC CCA CAT GCG TAT ATA ACT CAA GCT 403
Phe Asp Glu Thr Lys Tyr Lys Asn Pro His Ala Tyr Ile Thr Gln Ala
90 95 100

TGT TTT AAT GCA TTC GTC CAA CGT GGA TCC ATC GAT CCA CCA TCC GTA 451
Cys Phe Asn Ala Phe Val Gln Arg Gly Ser Ile Asp Pro Pro Ser Val
105 110 115

TCA GAA ATA CAA CTA ATG CAT AAT CTG GGT AAA CAT CTG AAT TCA ATG 499
Ser Glu Ile Gln Leu Met His Asn Leu Gly Lys His Leu Asn Ser Met
120 125 130

GAA CGT GTA GAA TGG CTG CGT AAA AAA CTG CAG GAT GTA CAT AAT TTT 547
Glu Arg Val Glu Trp Leu Arg Lys Lys Leu Gln Asp Val His Asn Phe
135 140 145

GTA GCT CTG GGT TAACTCGAGC AGATCCGGCT GCTAACAAAG CCCGAAAGGA 599
Val Ala Leu Gly
150

AGCTGAGTTG GCTGCTGCCA CCGCTGAGCA ATAACTAGCA TAACCCCTTG GGGCCCTTGG 659

GGCCTCTAAA CGGGTCTTGA GGGGTTTTTT GCTGAAAGGA GGAACTATAT CCGGATAA 717

(2) INFORMATION FOR SEQ ID NO:2:

(i) SEQUENCE CHARACTERISTICS:
(A) LENGTH: 153 amino acids
(B) TYPE: amino acid
(D) TOPOLOGY: linear

(ii) MOLECULE TYPE: protein

(xi) SEQUENCE DESCRIPTION: SEQ ID NO:2:

Met Ser Glu Thr Lys Pro Lys Tyr Asn Tyr Val Asn Asn Lys Glu Leu
1               5                   10                  15

Leu Gln Ala Ile Ile Asp Trp Lys Thr Glu Leu Ala Asn Asn Lys Asp
            20                  25                  30

Pro Asn Lys Val Val Arg Gln Asn Asp Thr Ile Gly Leu Ala Ile Met
        35                  40                  45

Leu Ile Ala Glu Gly Leu Ser Lys Arg Phe Asn Phe Ser Gly Tyr Thr
    50                  55                  60

Gln Ser Trp Lys Gln Glu Met Ile Ala Asp Gly Ile Glu Ala Ser Ile
65                  70                  75                  80

Lys Gly Leu His Asn Phe Asp Glu Thr Lys Tyr Lys Asn Pro His Ala
                85                  90                  95

Tyr Ile Thr Gln Ala Cys Phe Asn Ala Phe Val Gln Arg Gly Ser Ile
            100                 105                 110

Asp Pro Pro Ser Val Ser Glu Ile Gln Leu Met His Asn Leu Gly Lys
        115                 120                 125

His Leu Asn Ser Met Glu Arg Val Glu Trp Leu Arg Lys Lys Leu Gln
    130                 135                 140

Asp Val His Asn Phe Val Ala Leu Gly
145                 150

(2) INFORMATION FOR SEQ ID NO:3:

     (i) SEQUENCE CHARACTERISTICS:
          (A) LENGTH: 945 base pairs
          (B) TYPE: nucleic acid
          (C) STRANDEDNESS: single
          (D) TOPOLOGY: linear

    (ii) MOLECULE TYPE: DNA (genomic)

   (iii) HYPOTHETICAL: NO

   (iii) ANTI-SENSE: NO

     (v) FRAGMENT TYPE: internal

    (vi) ORIGINAL SOURCE:
          (A) ORGANISM: Bacteriophage T4/Homo sapiens

    (ix) FEATURE:
          (A) NAME/KEY: CDS
          (B) LOCATION: 101..787

    (xi) SEQUENCE DESCRIPTION: SEQ ID NO:3:

AGATCTCGAT CCCGCGAAAT TAATACGACT CACTATAGGG AGACCACAAC GGTTTCCCTC      60

TAGAAATAAT TTTGTTTAAC TTTAAGAAGG AGATATACAT ATG TCA GAA ACT AAG      115
                                            Met Ser Glu Thr Lys
                                              1               5

CCT AAA TAT AAT TAC GTA AAC AAT AAA GAG CTT TTA CAA GCT ATT ATT      163
Pro Lys Tyr Asn Tyr Val Asn Asn Lys Glu Leu Leu Gln Ala Ile Ile
                 10                  15                  20

GAT TGG AAA ACA GAA TTA GCA AAT AAT AAA GAC CCA AAT AAA GTA GTT      211
Asp Trp Lys Thr Glu Leu Ala Asn Asn Lys Asp Pro Asn Lys Val Val
             25                  30                  35

CGT CAG AAT GAT ACT ATC GGA TTA GCC ATT ATG CTT ATT GCA GAA GGC      259
Arg Gln Asn Asp Thr Ile Gly Leu Ala Ile Met Leu Ile Ala Glu Gly
         40                  45                  50

TTA TCT AAA CGT TTC AAC TTT TCA GGA TAC ACC CAG TCT TGG AAA CAA      307
Leu Ser Lys Arg Phe Asn Phe Ser Gly Tyr Thr Gln Ser Trp Lys Gln
     55                  60                  65

GAA ATG ATT GCA GAT GGT ATA GAA GCT TCT ATT AAG GGG CTT CAC AAT      355
Glu Met Ile Ala Asp Gly Ile Glu Ala Ser Ile Lys Gly Leu His Asn
 70                  75                  80                  85

TTT GAT GAA ACG AAA TAT AAA AAC CCA CAT GCG TAT ATA ACT CAA GCT      403
Phe Asp Glu Thr Lys Tyr Lys Asn Pro His Ala Tyr Ile Thr Gln Ala
                 90                  95                 100

TGT TTT AAT GCA TTC GTC CAA CGT ATT AAA AAA GAA CGT AAG GAA GTT      451
Cys Phe Asn Ala Phe Val Gln Arg Ile Lys Lys Glu Arg Lys Glu Val
            105                 110                 115

GCA AAG AAA TAT AGT TAC TTC GTT CAC AAT GTC TAT GAC AGC CGT GAC 499
Ala Lys Lys Tyr Ser Tyr Phe Val His Asn Val Tyr Asp Ser Arg Asp
        120                 125                 130

GAC GAT ATG GTT GCG TTA GTA GAT GAA ACT TTT ATT CAA GAC ATC TAT 547
Asp Asp Met Val Ala Leu Val Asp Glu Thr Phe Ile Gln Asp Ile Tyr
    135                 140                 145

GAT AAA ATG ACG CAT TAC GAA GAA TCA ACC TAT AGA ACA CCG GGG GCT 595
Asp Lys Met Thr His Tyr Glu Glu Ser Thr Tyr Arg Thr Pro Gly Ala
150                 155                 160                 165

GAA AAG AAA AGT GTT GTA GAT GAT TCT CCT AGT TTG GAT TTT TTA TAT 643
Glu Lys Lys Ser Val Val Asp Asp Ser Pro Ser Leu Asp Phe Leu Tyr
                170                 175                 180

GAG GCT AAC GAT GGA TCC GTT AAC GGT CCA TCC GTA TCA GAA ATA CAA 691
Glu Ala Asn Asp Gly Ser Val Asn Gly Pro Ser Val Ser Glu Ile Gln
            185                 190                 195

CTA ATG CAT AAT CTG GGT AAA CAT CTG AAT TCA ATG GAA CGT GTA GAA 739
Leu Met His Asn Leu Gly Lys His Leu Asn Ser Met Glu Arg Val Glu
        200                 205                 210

TGG CTG CGT AAA AAA CTG CAG GAT GTA CAT AAT TTT GTA GCT CTG GGT 787
Trp Leu Arg Lys Lys Leu Gln Asp Val His Asn Phe Val Ala Leu Gly
    215                 220                 225

TAACTCGAGC AGATCCGGCT GCTAACAAAG CCCGAAAGGA AGCTGAGTTG GCTGCTGCCA 847

CCGCTGAGCA ATAACTAGCA TAACCCCTTG GGGCCCTTGG GGCCTCTAAA CGGGTCTTGA 907

GGGGTTTTTT GCTGAAAGGA GGAACTATAT CCGGATAA 945

(2) INFORMATION FOR SEQ ID NO:4:

(i) SEQUENCE CHARACTERISTICS:
(A) LENGTH: 229 amino acids
(B) TYPE: amino acid
(D) TOPOLOGY: linear

(ii) MOLECULE TYPE: protein

(xi) SEQUENCE DESCRIPTION: SEQ ID NO:4:

Met Ser Glu Thr Lys Pro Lys Tyr Asn Tyr Val Asn Asn Lys Glu Leu
1               5                   10                  15

Leu Gln Ala Ile Ile Asp Trp Lys Thr Glu Leu Ala Asn Asn Lys Asp
            20                  25                  30

Pro Asn Lys Val Val Arg Gln Asn Asp Thr Ile Gly Leu Ala Ile Met
        35                  40                  45

Leu Ile Ala Glu Gly Leu Ser Lys Arg Phe Asn Phe Ser Gly Tyr Thr
    50                  55                  60

Gln Ser Trp Lys Gln Glu Met Ile Ala Asp Gly Ile Glu Ala Ser Ile
65                  70                  75                  80

Lys Gly Leu His Asn Phe Asp Glu Thr Lys Tyr Lys Asn Pro His Ala
                85                  90                  95

Tyr Ile Thr Gln Ala Cys Phe Asn Ala Phe Val Gln Arg Ile Lys Lys
            100                 105                 110

Glu Arg Lys Glu Val Ala Lys Lys Tyr Ser Tyr Phe Val His Asn Val
        115                 120                 125

Tyr Asp Ser Arg Asp Asp Asp Met Val Ala Leu Val Asp Glu Thr Phe
    130                 135                 140

Ile Gln Asp Ile Tyr Asp Lys Met Thr His Tyr Glu Glu Ser Thr Tyr
145                 150                 155                 160

Arg Thr Pro Gly Ala Glu Lys Lys Ser Val Val Asp Asp Ser Pro Ser
                165                 170                 175

Leu Asp Phe Leu Tyr Glu Ala Asn Asp Gly Ser Val Asn Gly Pro Ser
            180                 185                 190

Val Ser Glu Ile Gln Leu Met His Asn Leu Gly Lys His Leu Asn Ser
        195                 200                 205

Met Glu Arg Val Glu Trp Leu Arg Lys Lys Leu Gln Asp Val His Asn
    210                 215                 220

Phe Val Ala Leu Gly
225

(2) INFORMATION FOR SEQ ID NO:5:

     (i) SEQUENCE CHARACTERISTICS:
          (A) LENGTH: 27 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

   (iii) ANTI-SENSE: NO

     (v) FRAGMENT TYPE: internal


    (xi) SEQUENCE DESCRIPTION: SEQ ID NO:5:

GAGGTGCATA TGTCAGAAAC TAAGCCT                                          27

(2) INFORMATION FOR SEQ ID NO:6:

     (i) SEQUENCE CHARACTERISTICS:
          (A) LENGTH: 40 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

   (iii) ANTI-SENSE: NO

     (v) FRAGMENT TYPE: internal


    (xi) SEQUENCE DESCRIPTION: SEQ ID NO:6:

GGTCGGATCC ATCGTTAGCG TTAGCCTCAT ATAAAAAATC                            40

(2) INFORMATION FOR SEQ ID NO:7:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(v) FRAGMENT TYPE: internal

(xi) SEQUENCE DESCRIPTION: SEQ ID NO:7:

GGTCGGATCC TACGTTGGAC GAATGCAT 28

(2) INFORMATION FOR SEQ ID NO:8:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(v) FRAGMENT TYPE: internal

(xi) SEQUENCE DESCRIPTION: SEQ ID NO:8:

GAGTGGATCC ATCGATCCAC CATCCGTATC AGAAATACAA CT 42

(2) INFORMATION FOR SEQ ID NO:9:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(v) FRAGMENT TYPE: internal

(xi) SEQUENCE DESCRIPTION: SEQ ID NO:9:

GAGTGGATCC GTTAACGGTC CATCCGTATC AGAAATACAA CT 42

(2) INFORMATION FOR SEQ ID NO:10:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(v) FRAGMENT TYPE: internal

(xi) SEQUENCE DESCRIPTION: SEQ ID NO:10:

TCTACTCGAG TTAACCCAGA GCTACAAAAT TATG 34

(2) INFORMATION FOR SEQ ID NO:11:

(i) SEQUENCE CHARACTERISTICS:
(A) LENGTH: 34 amino acids
(B) TYPE: amino acid
(C) STRANDEDNESS: single
(D) TOPOLOGY: unknown

(ii) MOLECULE TYPE: protein

(iii) HYPOTHETICAL: NO

(iii) ANTI-SENSE: NO

(vi) ORIGINAL SOURCE:
(A) ORGANISM: Homo sapiens

(xi) SEQUENCE DESCRIPTION: SEQ ID NO:11:

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe

(2) INFORMATION FOR SEQ ID NO:12:

(i) SEQUENCE CHARACTERISTICS:
(A) LENGTH: 36 amino acids
(B) TYPE: amino acid
(C) STRANDEDNESS: single
(D) TOPOLOGY: unknown

(ii) MOLECULE TYPE: protein

(iii) HYPOTHETICAL: NO

(iii) ANTI-SENSE: NO

(vi) ORIGINAL SOURCE:
(A) ORGANISM: Homo sapiens

(xi) SEQUENCE DESCRIPTION: SEQ ID NO:12:

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe Val Ala
        35

(2) INFORMATION FOR SEQ ID NO:13:

(i) SEQUENCE CHARACTERISTICS:
    (A) LENGTH: 38 amino acids
    (B) TYPE: amino acid
    (C) STRANDEDNESS: single
    (D) TOPOLOGY: unknown

(ii) MOLECULE TYPE: protein

(iii) HYPOTHETICAL: NO

(iii) ANTI-SENSE: NO

(vi) ORIGINAL SOURCE:
    (A) ORGANISM: Homo sapiens

(xi) SEQUENCE DESCRIPTION: SEQ ID NO:13:

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe Val Ala Leu Gly
        35

## Description

The present invention relates to variants of parathyroid hormone (PTH), a process for their production, pharmaceutical preparations comprising them and their use as a pharmaceutical. Compounds of this type are well-known, e.g., see WO9211286.

The term "PTH" as used herein refers to any genetically encoded form of parathyroid hormone, including the mature form containing 84 amino acids of a given vertebrate PTH species, e.g human, porcine, rat, bovine, chicken, and fragments thereof as well as analogues and derivatives thereof having PTH-like activity. The position of each amino acid involved in the PTH sequence is numbered according to the internationally accepted procedure: For consistency and as is conventional, in the following description, the same numbering system will be applied to the amino acids of the PTH sequence independently of the substitution pattern in the molecule.

More particularly, the present invention provides a PTH compound having PTH-like activity of formula 1
Ser-Val-Ser-Glu-Ile-Gln-Leu-R₁-His-R₂-R₃-Gly-R₄-His-Leu-R₅-R₆-R₇-R₈-Arg-Val-Glu-Trp-Leu-Arg-R₉-Lys-Leu-Gln-Asp-Val-His-R₁₀-R₁₁-(R₁₂)ₙ-X
wherein
R₁ is Met or Leu;
R₂ is Asn, Asp, Gly, Gln, Glu, His, Ser, Thr or Tyr;
R₃ is Leu or Lys;
R₄ is Lys or D- or L-Ala, -Cys, -Gln, -Ile, -Asn, -Trp, -Asp, -Val, -Thr, -Ser, -Tyr, -Met, -Leu or -Gly;
R₅ is Asn, Gln, D-Gln or D- or L-Lys, -Ser. -Leu. -Ala or -Gly;
R₆ is Ser, Asp, Ala. Glu, Gln, Phe, His, Ile or Lys;
R₇ is Gln
R₈ is Glu, Arg, Ala. Val, Tyr, Ser, Lys, Met, His, Gly, Pro, Asn or Ile;
R₉ is Lys, Gln or Arg;
R₁₀ is Asn, Thr, D-Thr or D- or L-Ser, -Leu, -Gly, -Gln, -Arg, -Pro, -Asp, -Ile or -Lys;
R₁₁ is Phe or Ala;
R₁₂ is Val, Val-Ala, Val-Ala-Leu or Val-Ala-Leu-Gly;
n is 0 or 1; and
X is OH, ORₐ, NH₂ or NR_{b}R_{c} wherein Rₐ is C₁₋₄alkyl, one of R_{b} and R_{c} is H and the other is C₁₋₆alkyl, the compound optionally comprising at least one radical selected from a L- or D-α-amino acid, C₂₋₆alkoxy-carbonyl and optionally substituted C₁₋₈ alkyl, C₂₋₈alkenyl, C₂₋₈ alkynyl, aralkyl, aralkenyl or C₃₋₆ cycloalkyl-C₁₋₄alkyl and attached to its terminal amino group, and/or
the compound optionally comprising at least one radical selected from C₂₋₆alkoxycarbonyl and optionally substituted C₁₋₈alkyl, C₂₋₈alkenyl, C₂₋₈alkynyl, aralkyl, aralkenyl or C₃₋₆cycloalkyl-C₁₋₄alkyl attached to one or more side chain amino groups of the compound, in free form or in salt or complex form.

Hereinafter, these compounds will be referred to as compounds of the invention.

"Natural amino acids" refer to those well known in the art. They are listed and standard abbreviations are provided in the U.S.P.T.O. publication, Trademark Official Gazette, published May 15, 1990, P. 33 at 46. These amino acids and abbreviations are specifically incorporated herein by reference.

The natural amino acids are shown below:

| | | |
|---|---|---|
| A | Ala | alanine |
| D | Asp | aspartic acid |
| E | Glu | glutamic acid |
| F | Phe | phenylalanine |
| G | Gly | glycine |
| H | His | histidine |
| I | Ile | isoleucine |
| K | Lys | lysine |
| L | Leu | leucine |
| M | Met | methionine |
| N | Asn | asparagine |
| Q | Gln | glutamine |
| R | Arg | arginine |
| S | Ser | serine |
| T | Thr | threonine |
| V | Val | valine |
| W | Trp | tryptophane |
| Y | Tyr | tyrosine |

By amino acid in protected form is meant a natural amino acid having e.g. a side chain including an heteroatom such as 0, S or N which can be protected with an 0-, S- or N-protecting group. The N-terminus of the PTH compounds of the invention may also be in protected form.

N-protecting groups as may be present on the N-terminus or side chain amino groups of amino acid units include such groups as e.g. disclosed in "Protective Groups in Organic Synthesis", T. W. Greene, J.Wiley & Sons NY (1981), 219-287, for example acyl such as formyl, acetyl, trifluoroacetyl, methoxysuccinyl, hydroxysuccinyl or benzoyl optionally substituted on the phenyl ring with e.g. p-methoxycarbonyl, p-methoxy, p-nitro or p-phenylsulfonamidocarbonyl; alkoxycarbonyl such as t-butyloxycarbonyl, isobutyloxycarbonyl or methoxycarbonyl; allyloxycarbonyl; trityl; 2,2,5,7,8-pentamethyl-chroman-6-sulfonyl; arylmethoxycarbonyl such as 9-fluorenylmethoxycarbonyl or benzyloxy carbonyl optionally substituted on the phenyl ring with p-methoxy, p-nitro, o- or p-chloro, m-phenyl or 3,4-dimethyl; arylmethyl such as benzyl optionally ring substituted with p-methoxy, p-nitro or p-chloro; or arylsulfonyl such as phenylsulfonyl optionally ring substituted with p-methyl or p-methoxy, or naphthylsulfonyl optionally ring substituted with e.g. amino or di(C₁₋₄alkyl)amino.

0-protecting groups of 0-containing side chains are e.g. as described in "The Peptides", 3, (1981), E. Gross and J. Meienhofer (Ed.). For aliphatic hydroxy functionalities, suitable 0-protecting groups are e.g. as disclosed in the above reference in "Protection of the Hydroxy Group", J.M. Stewart, 169-201 and include the benzyl, t.-butyl and methyl groups. For aromatic hydroxy functionalities, suitable 0-protecting groups include the benzyl, t.-butyl, methyl, tosyl and benzyloxycarbonyl groups. 0-protecting groups for carboxy functionalities on amino acid side chains are well known ester groups and described e.g. in "The Peptides", 3, 101-135 (11Carboxyl Protecting Groups" by R.W. Roeske) and include the methyl, ethyl, t.-butyl and benzyl groups. S-protecting groups for thiol functionalities on amino acid side chains are known and described e.g. in "The Peptides", 3, 137-167 ("Sulfhydryl Group Protection" by R.G. Hiskey). Examples include the methyl, t.-butyl, benzyl, p-methoxyphenylmethyl, ethylamino-carbonyl and benzyloxycarbonyl groups.

According to the invention the PTH compounds may bear on their terminal amino group at least one radical selected from a L- or D-α-amino acid, C₂₋₆alkoxycarbonyl and optionally substituted C₁₋₈alkyl, C₂₋₈alkenyl, C₂₋₈alkynyl, aralkyl, aralkenyl or C₃₋₆cycloalkyl-C₁₋₄alkyl, and/or on one or more side chain amino group(s) at least one radical selected from C₂₋₆alkoxycarbonyl and optionally substituted C₁₋₈alkyl, C₂₋₈alkenyl, C₂₋₈alkynyl, aralkyl, aralkenyl or C₃₋₆cycloalkyl-C₁₋₄alkyl. When such a group is attached to a side chain amino group, it is preferably on the s-amino group of a Lys unit. Preferred substituents for the alkyl, alkenyl, aralkyl, aralkenyl or cycloalkylalkyl group are hydroxy, amino and for the aryl moiety also halogen and/or C₁₋₄alkoxy. The alkyl group or alkyl moiety may be linear or branched and optionally interrupted by 0, S or N. Preferably any C₁₋₈alkyl, C₂₋₈alkenyl, aralkyl, aralkenyl or C₃₋₆cycloalkyl-C₁₋₄alkyl on the amino group of a PTH compound, is non-substituted. Examples for C₁₋₈alkyl are C₁₋₆alkyl, preferably methyl, ethyl, propyl, isopropyl, butyl, isobutyl, t.-butyl; for C₂₋₈alkenyl, C₂₋₄alkenyl, preferably allyl; for C₂₋₈alkynyl, C₂₋₄alkynyl, preferably prop-2-ynyl; for aralkyl, phenyl or benzyl; for aralkenyl, styryl; for C₃₋₆cycloalkyl-C₁₋₄alkyl, cyclohexyl-methyl. C₂₋₆alkoxycarbonyl is preferably formyl or acetyl. Suitable examples of D- or L-α-amino acids attached to the N-terminus include e.g. D- or L- Pro, Ala. Preferred substituents when present are alkyl, alkynyl, alcoxycarbonyl or a D- or L-α-amino acid attached to the N-terminus of the PTH compound and/or at least one alkyl or alkoxycarbonyl attached to one or more side chain amino groups.

Examples of preferred compounds according to the invention are [Leu⁸, Gln¹⁸, Thr³³, Ala³⁴]PTH(1-x'), [Leu⁸, Ala¹⁶, Gln¹⁸, Thr³³. Ala³⁴]PTH (1- x'), [Leu⁸, Asp¹⁰, Lys¹¹, Ala¹⁶, Gln¹⁸, Thr³³, Ala³⁴]PTH (1-x'), [Leu⁸, Asp¹⁰, Lys¹¹, Ala¹⁶, Gln¹⁸]PTH(1- x'), [Leu⁸, Ala¹⁶, Gln¹⁸, Ala¹⁹]PTH(1- x'), [Leu⁸, Asp¹⁰, Lys¹¹, Gln18]PTH(1- x'), [Leu⁸, Asp¹⁰, Lys¹¹, Ala¹⁶, Gln¹⁸, Ala¹⁹]PTH(1- x'), [Leu⁸, Ala¹⁶, Gln¹⁸, Ala¹⁹, Thr³³, Ala³⁴]PTH(1- x'), and [Leu⁸, Asp¹⁰, Lys¹¹, Gln¹⁸, Thr³³, Ala³⁴]PTH, wherein x' is an integer from 34 to 38, particularly 34, 36, 37 or 38. Especially PTH (1- x') is hPTH (1- x').

The compounds of the invention may exist e.g. in free form, salt form or in the form of complexes thereof. Acid addition salts may be formed with e.g. organic acids, polymeric acids and inorganic acids. Such acid addition salt forms include e.g. the hydrochlorides and the acetates. Complexes are e.g. formed from the compound of the invention on addition of inorganic substances, e.g. inorganic salts or hydroxides such as Ca- and Zn-salts, and/or an addition of polymeric organic substances.

The present invention also provides a process for the production of the compounds of the invention. They may be prepared in a stepwise manner either in solution or using the solid phase synthesis process or genetic engineering.

The compounds of the invention may be produced for example as follows:
a) removing at least one protecting group which is present in a compound of the invention in protected form; or
b) linking together by an amide bond two peptide fragments, the peptide fragments being such that the desired amino acid sequence of the desired compound is obtained, and then effecting optionally stage a) of the process,
c) for the production of a PTH compound comprising at least one radical selected from D- or L-α-amino acid attached to the N-terminus and C₂₋₆alkoxycarbonyl and optionally substituted C₁₋₈alkyl, C₂₋₈alkenyl, C₂₋₈alkynyl, aralkyl, aralkenyl or C₃₋₆cycloalkyl-C₁₋₄alkyl attached to the terminal amino group and/or to a side chain amino group, introducing such a radical in a PTH compound in protected or unprotected form and free from such a radical, and if necessary carrying out process step a);
and recovering the compounds thus obtained in free form, in salt form or in complex form.

Process steps a), b) and c) may be effected in analogy with known methods, e.g. as known in the art of peptide chemistry or as described in the following examples. Where desired, in these reactions, protecting groups which are suitable for use in peptides may be used for functional groups which do not participate in the reaction. The term protecting group may also include a polymer resin having functional groups.

Process step c) may be carried out in analogy with known methods, e.g. alkylating methods. In a preferred embodiment of the invention, the alkylation is performed under reductive conditions, e.g. using a corresponding aldehyde or ketone. It may be performed for example in the presence of NaBH₃CN, preferably at an acidic pH, e.g. from 5 to 7. The temperature of the reaction may be e.g. from -20° C to 100°C. It may be advantageous to carry out the reaction in an inert solvent, e.g. water, an alcohol, dioxane or DMF or a mixture thereof. A D- or L-α-amino acid may also be attached to the N-terminus in accordance with known procedure.

The peptide fragment used as starting material in process step b) may be substituted on the N-terminus and/or on a side chain amino group by a radical selected from C₂₋₆alkoxycarbonyl, optionally substituted C₁₋₈alkyl, C₂₋₈alkenyl, C₂₋₈ alkynyl, aralkyl, aralkenyl or C₃₋₆cycloalkyl-C₁₋₄alkyl or it may bear a D- or L-α-amino acid on the N-terminus.

The compounds of the invention or fragments thereof comprise natural α-amino acid units and may also be prepared using recombinant technology.

According to a preferred embodiment of the invention there is also provided a process for the production of a compound of the invention, in which a fusion protein comprising an N-terminal bacteriophage T4 gene 55 polypeptide having the desired compound linked to the C-terminal thereof, is expressed in bacterial cells and where required, introducing a C₂₋₆alkoxy carbonyl or optionally substituted C₁₋₈alkyl, C₂₋₈alkenyl, C₂₋₈alkynyl, aralkyl, aralkenyl or C₃₋₆cycloalkyl-C₁₋₄alkyl on the terminal and/or side chain amino group of the compound in protected or unprotected form and, if required, removing the protecting group present in the protected form.

The gene 55 polypeptide may comprise the complete gp55 protein of bacteriophage T4 (188 amino acid residues) which is described in Gram, H. and Rüger, R; 1985; The EMBO Journal, 4, (1), pp 257-264. Alternatively a fragment, preferably an N-terminal fragment of the gp55 protein may be used. For example, a gp55 protein fragment comprising the first 112 N-terminal amino acid residues of the protein may be used. Typically, however, the gene 55 polypeptide comprises at least the first 25 N-terminal residues of the gp⁵⁵ protein.

Conveniently the gene 55-PTH fusion protein also comprises a cleavable linker between the gene 55 polypeptide and the desired compound. Preferably the cleavable linker is chemically cleavable and more preferably contains the amino acid sequences Asp-Pro-Pro or Asn-Gly-Pro.

In a further aspect the invention provides a nucleotide sequence coding for a fusion protein comprising an N-terminal bacteriophage T4 gene 55 polypeptide and a desired compound of the invention linked to the C-terminal thereof.

The nucleotide sequence is preferably a DNA sequence suitable for expression in bacteria. The sequence typically contains nucleotides coding for a cleavable linker between the gene 55 and desired compound coding sequences.

The invention also provides a bacterial expression vector comprising a DNA sequence coding for the fusion protein.

The expression vector typically contains, in addition to the fusion protein coding sequence, appropriate expression control sequences including a suitable promoter, an operator and a ribosome binding site and other appropriate regulatory sequences. Usually, also the expression vector contains one or more selectable markers.

Any suitable promoter may be used, such as the TrpE, λPl, λPr, lac or Tac promoter. Preferably the promoter is the bacteriophage T7 promoter and the expression vector is a plasmid. For E. coli expression an Rl or Col-E1 plasmid-derived vector may be used. For example, expression vector pET 17B, obtainable from Novagen, or similar expression vectors may be used.

In another aspect the invention provides bacterial host cells transformed with a fusion protein coding sequence or an expression vector as described above. Any suitable bacterial host may be used, though preferably the host is E. coli. For example, E. coli strain BL21 (DE3) Lys E and similar strains may be used.

Advantageously the fusion protein accumulates within the host cells in the form of insoluble inclusion bodies, and thereby facilitates recovery of the fusion protein product from the cells. In order to recover the desired polypeptide product from the fusion protein, the protein of the inclusion bodies is solubilised, and the desired polypeptide product cleaved from the fusion protein. Any suitable solubilisation treatment may be used, including acid treatment, e.g. at about pH 2 to 4, preferably with acid at about pH 2.5, or treatment with a denaturing agent, e.g. a mild denaturating agent such as guanidine hydrochloride. In addition it may be necessary to remove one or more unwanted N-terminal amino acid residues from the product which remain after cleavage.

Thus in a further aspect the invention provides a process for the production of a desired compound of the invention, in a bacterial host, the process comprising: causing transformed bacterial host cells as defined above to express the fusion protein in the form of inclusion bodies; isolating the inclusion bodies; solubilizing the inclusion bodies, and cleaving the desired polypeptide from the fusion protein.

In the case of PTH compounds, it is not normally necessary to carry out carefully controlled denaturation and renaturation procedures to obtain PTH products in active form, e.g. having PTH-like activity. The medium-sized polypeptide products may be obtained in active form merely by neutralising the acid conditions used for solubilisation. Solubilisation and cleavage of the fusion protein may be carried out in a single step.

The invention also includes a fusion protein comprising an N-terminal bacteriophage T4 gene 55 polypeptide and, linked to the C-terminal thereof, a desired compound of the invention.

Using the processes and vectors of the invention it has been found that it is possible to produce high levels of PTH products. In E. coli, we have found that up to about 50% of the total protein expressed is the desired fusion protein. Also, the fusion protein forms inclusion bodies which are easy to separate from the other material of the cells. Moreover the inclusion bodies often consist of at least about 90% of the desired fusion protein which can significantly reduce the amount of purification that is required. Further, expressing the PTH product, in the form of the fusion protein can substantially decrease endogenous processing of the polypeptide in the bacterial cell, allowing homogenous products to be obtained.

Preferably the cleavable linker contains chemically cleavable amino acids adjacent the N-terminus of the desired polypeptide, to permit the desired polypeptide to be cleaved from the fusion protein by simple chemical means. Preferably the desired compound does not contain the chemically cleavable groups.

Preferred chemically cleavable linkers contain the amino acids Asp-Pro-Pro or Asn-Gly-Pro. The Asp-Pro or Asn-Gly bonds may be chemically cleaved using acid conditions e.g. formic acid (normally about pH 2.5) or hydroxylamine respectively. The dipeptides Pro-Pro or Gly-Pro that remain on the N-terminus of the desired polypeptide may then be removed using a suitable dipeptidyl-peptidase. For example the L. lactis X-Pro dipeptidyl-peptidase EC 3.4.14.5 can be used. This peptidase is described in Nardi et al; 1991; Applied and Environmental Microbiology, 57, p45 to 50.

The compound of the invention may be purified following cleavage from the fusion protein. HPLC purification techniques may be used. Purification may be carried out before removal of any unwanted N-terminal amino acid residues.

The process of the invention is further illustrated in the examples 25 to 31 which however only relate to fusion proteins comprising (1-38)PTH which are not comprised in the invention. SEQ ID No.1 shows the amino acid sequence and corresponding DNA sequence of a truncated gp55-Asp-Pro-ProhPTH(1-38)OH fusion protein as cloned into the plasmid pET17B, and SEQ ID No.3 shows the amino acid sequence and corresponding DNA sequence of a full length gp55-Asn-Gly-Pro-hPTH(1-38)OH fusion protein as cloned into the plasmid pET17B.

In the following Examples all temperatures are in 0°C. The following abbreviations are employed. In the present specification the term PTH without any further indication includes the carboxy terminus as well as a carboxamide terminus.
- BOC: tert.Butyloxycarbonyl
- DMF: dimethylformamide
- DCM: dichloromethane
- Fmoc: 9-fluorenylmethoxycarbonyl
- HOBt: 1-hydroxybenzotriazole
- TFA: trifluoroacetic acid
- Trt: trityl = triphenylmethyl
- RP-HPLC: reverse phase high performance liquid chromatography
- r.t.: room temperature
- DNP: Dinitrophenyl
- Tos: Tosyl
- DIPC: Diisopropylcarbodiimide
- Pmc: 2,2,5,7,8-Pentamethylchroman-6-sulfonyl
- IP: Isopropyl
- For: Formyl
- Cpe: 1-amino-eyclopentane-1-carboxylic acid
- Cpp: 1-amino-cyclopropane-1-carboxylic acid
- Cha: cyclohexylalanine
- tBu: t.butyl

### Comparative EXAMPLE 1: N^{α}--Isopropyl hPTH(1-34) NH₂

This peptide is assembled in a stepwise manner on a polystyrene based resin support. The Boc-group is used for protection of the alpha amino-group and side-chain functional groups are protected as follows: Lys(2-chlorobenzyloxycarbonyl), Ser(benzyl), Thr(benzyl), Glu(benzyl), Asp(benzyl), Met(O), His(DNP), Arg(Tos), and Trp(HCO). Other residues are left unprotected.

Amino-4-methylphenyl-methyl-co(polystyrene-divinylbenzene = MBHA resin) (0,7 mmol/g) is subjected to the following cycle, steps (1) to (7), of treatments:
(1) DCM
(2) trifluoracetic acid (50 %) in DCM
(3) DCM
(4) diisopropylethylamine (10 %) in DMF
(5) DMF
(6) preformed symmetrical anhydride (1.4 mmol per g starting resin) of Boc-amino acid in DMF
(7) DMF

Volumes of washes and reagents are from 5 to 20 ml per gram of starting resin. Each step is repeated as many times as necessary for either complete reaction of the resin (steps 2, 4, 6) or complete displacement of the previous reagent from the resin (steps 1, 3, 5, 7). Samples of resin are removed after each cycle and checked for completeness of the coupling reaction by a colorimetric test for residual amino groups using ninhydrin.

Symmetrical anhydrides of Boc-amino acids are prepared just prior to use by reacting Boc-amino acid (2,8 mmol per g resin) and DCCI (1,4 mmol per g resin) in DCM, containing DMF in amounts sufficient for complete dissolution of the Boc-amino acid. The mixture is filtered, more DMF is added to the filtrate which is concentrated by evaporation of the volatile components at a temperature not exceeding 15°C and the resulting solution is used in step (6).

The cycle of reactions, (1) to (7), is repeated for each amino acid residue such as to provide the sequence of the title compound except for Boc-Gln-OH and Boc-Arg(Tos)-OH, which are coupled in step (6) as their preformed 1-hydroxybenzo-triazole esters in DMF.

The fully assembled peptide resin is treated twice with 20 ml of thiophenol A in DMF at r.t. for 1 hour and washed with DMF, methanol and dichloromethane. The peptide resin is again subjected to steps (1) to (5) followed by the addition of 0.5 ml of acetone, 84 mg of sodium cyanoborohydride and 0.2 ml of acetic acid in 20 ml of DMF per gram of resin. After 16 hours at RT the resin is washed with DMF and dichloromethane and dried.

The peptide resin is treated with liquid HF, dimethyl sulfide, p-cresol and ethane-1,2-dithiol according to the 'low-high' procedure which is described, e.g., in International Journal of Peptide and Protein Research, vol.26, page 262-273 (1985). After removal of the volatile components and washing with ethylacetate the residue is extracted with several portions of 1%-acetic acid, filtered and the filtrate lyophilized. The lyophilized product is purified by repeated reversed-phase chromatography on a column of octadecyl-silica using a gradient of acetonitrile in 2%-phosphoric acid or in 20 mM tetramethylammonium phosphate. Fractions containing the pure compound are combined, filtered through a weakly basic ion-exchange resin in the acetate form and the filtrate lyophilized to give the title compound. MS (Ion-Spray): 4160.

### Comparative EXAMPLE 2: [Lys (N^{ε}-Isopropyl)^{26,27}]hPTH(1-34) NH₂

The peptide is assembled as described for Example 1 but using Lys(Fmoc) for incorporation in position 13 and Fmoc-Ser(tBu) in terminal position 1.

The fully assembled peptide resin is treated twice with 20 ml of thiophenol 5% in DMF at r.t. for 1 hour and washed with DMF, methanol and dichloromethane. The dry resin is treated with liquid HF as described for Example 1. The cleavage product (350 mg) is suspended in a mixture of methanol (5 ml), phosphate buffer pH 5.0 (5 ml), sodium cyanoborohydride (48 mg) and acetone (0.28 ml). After 16 hours at RT the reaction mixture is filtered and the filtrate evaporated. The residue is suspended in 20%-piperidine in DMF for 15 minutes, diluted with 20 volumes of ether and filtered. The residue is dissolved in 100 ml of water and acetic acid added to pH = 3, filtered and the filtrate lyophilized. The lyophilizate is purified by reversed-phase chromatography on a column of octadecyl-silica using a gradient of acetonitrile in 2%-phosphoric acid. Fractions containing the pure compound are combined, filtered through a weakly basic ion-exchange resin in the acetate form and the filtrate lyophilized to give the title compound. MS (Ion Spray): 4205.6.

### Comparative EXAMPLE 3: N^{α}-Isopropyl[Lys (N^{ε}-Isopropyl)^{13,26,27}]hPTH-(1-38)-OH

### a) Solid phase peptide synthesis

The peptide is synthesized in a stepwise manner on a polystyrene based resin support. The alpha-amino group is protected by Fmoc and the side-chain functional groups as followed: Asp(OtBu), Glu(OtBu), His(Trt), Lys(Boc), Asn(Trt), Gln(Trt), Arg(Pmc) and Ser(tBu). Other amino acids are left unprotected.

Fmoc-Gly esterified to 4-Hydroxymethyl-phenoxymethylco(polystyrene-1%-divinylbenzene), 0.5 mmol/g, is used as starting material for the stepwise solid phase synthesis, which consists of repetitive cycles of alpha-amino deprotection, washing, coupling (attachment of new amino acid) and washing. A three to five fold excess of Fmoc-amino acids is coupled as preformed HOBt-esters using DIPC. Fmoc-Arg(Pmc), Fmoc-Asn(Trt) and Fmoc-Gln(Trt) are coupled as symmetrical anhydrides using DIPC. After complete assembly of the peptide chain the Fmoc-protecting group of Ser¹ is removed by 20% piperidine/DMF. Cleavage of the peptide from the polystyrene resin and removal of all side chain protecting groups is obtained by treatment of the peptide resin with a mixture of 10% ethanedithiol, thioanisole, thiocresole and water in 90% TFA for three hours at room temperature. The resin particles are filtered off and washed with some TFA. The product is precipitated from the combined filtrates by addition of ether, filtered and dried. The product is purified by chromatography on a C-18 silica column using a gradient of acetonitrile in 2% H₃P0₄/water. Fractions containing the pure compound are collected, filtered through an anion-exchange resin (Biorad, AG 4-X4, 100-200mesh acetate form) and lyophilized to give hPTH-(1-38)-OH.

### b) Alkylation

40 mg (9 µmol) hPTH-(1-38)-OH(4458.2 g/mol) are dissolved in 1.2 ml methanol, 1.2 ml phosphate buffer (Merck no. 9887 adjusted to pH 5.0), 45 mg (716 nml) NaBH₃CN (62.84 g/mol) and 0.8 ml (11 mmol) acetone (73.52 ml/mol). The reaction is monitored by RP-HPLC on a C-18 silica column and finished after 15 hours at room temperature. The reaction mixture is diluted with water and chromatographied on a C-18 silica column using a gradient of acetonitrile in 2% H₃P0₄/water. Fractions containing the pure compound are collected, filtered through an anion-exchange resin (acetate form) and lyophilized to give the title compound as polyacetate, polyhydrate.
[a]_{D}²⁰ = 5.7° (c= 0.317 in 95% AcOH).
MS(Ion-Spray): 4626

### Comparative EXAMPLE 4: Nα-Methyl[Ala¹-]PTH-(1-38)-OH

The peptide chain is assembled in the same manner as in Comparative example 3a. At position 1 instead of Fmoc-Ser(tBu)-OH the unnatural amino acid Fmoc-N^{α}-Methyl-Ala-0H is coupled to the peptide resin. Cleavage, deprotection and purification is performed as in example 3a. MS (Ion Spray) = 4455,91

### Comparative EXAMPLE 5: [Ala¹,Ala³,Leu⁸,Gln¹³,Ala¹⁶ Gln¹⁸,Ala¹⁹,Phe²³,His²⁵ His²⁶,Leu²⁷,Thr³³,Ala³⁴]hPTH (1-34)OH

This peptide is prepared in analogy with the procedure of comparative Example 3a. Instead of Fmoc-Gly esterified to 4-hydroxymethyl-phenoxymethyl-co(polystyrene-divinylbenzene) the appropriate Fmoc-amino acid, e.g. Fmoc-Ala, Fmoc-Phe, Fmoc-D-Ala, Fmoc-D-Phe, etc. is used. Additionally the side-chain functional groups are protected as follows: Thr(t.-butyl), Trp(Boc) and Tyr(t.-butyl).

By repeating the procedure disclosed in Examples 3 and 5 but using the appropriate starting materials, the following compounds may be obtained:
Example 1: [Leu⁸, Asp¹⁰, Ala¹⁶, Gln¹⁸, Thr³³]hPTH (1-34)OH
Example 2: [Leu⁸, Lys¹¹, Gln¹⁸]hPTH (1-36)OH
Example 3: [Leu⁸, Gln¹⁸, Thr³³, Ala³⁴]-hPTH (1-34)OH (IS-MS: 4007)
Example 4: [Leu⁸, Ala¹⁶, Gln¹⁸, Ala¹⁹, Thr³³, Ala³⁴]hPTH (1-34)OH
Example 5: [Leu⁸, Ala¹⁶, Gln¹⁸, Thr³³, Ala³⁴]hPTH (1-34)OH (IS-MS: 3965)
Example 6: [Leu⁸, Asp¹⁰, Lys¹¹, Gln¹⁸]hPTH (1-36)OH
Comparative Example 7: [Leu⁸, Gln¹⁶, Asp¹⁷, Leu¹⁸, Arg¹⁹, Thr³³, Ala³⁴]-hPTH (1-34)OH
Example 8: [Leu⁸, Asp¹⁰, Lys¹¹, Gln¹⁸, Thr³³, Ala³⁴]hPTH (1-34)OH (IS-MS: 4022)
Comparative Example 9: [Leu⁸, Asp¹⁰, Lys¹¹, Gln¹⁶, Asp¹⁷, Leu¹⁸, Arg¹⁹, Thr³³, Ala³⁴]hPTH (1-34)OH (IS-MS: 4077)
Example 10: [Leu⁸, Asp¹⁰, Lys¹¹, Ala¹⁶, Gln¹⁸, Ala¹⁹]hPTH (1-36)OH (IS-Ms: 4181)
Example 11:[Leu⁸, Ala¹⁶, Asp¹⁷, Gln¹⁸, Ala¹⁹]hPTH (1-36)OH (IS-MS: 4193)
Example 12:[Leu⁸, Ala¹⁶, Ala¹⁷, Gln¹⁸, Ala¹⁹]hPTH (1-36)OH (IS-MS: 4149)
Example 13:[Leu⁸, Ala¹⁷, Gln¹⁸, Ala¹⁹]hPTH (1-36)OH
Example 14: [Leu⁸, Asp¹⁰, Lys¹¹, Ala¹⁶, Gln¹⁸, Thr³³, Ala³⁴]hPTH (1-34)OH (IS-MS: 3980)
Example 15: [Leu⁸, Asp¹⁰, Lys¹¹, Ala¹⁶, Gln¹⁸]hPTH (1-36)OH (IS-MS: 4240)
Example 16: [Leu⁸, Asp¹⁰, Lys¹¹, Ala¹⁶, Gln¹⁸, Ala¹⁹, Thr³³, Ala³⁴]hPTH (1-34)OH (IS-MS: 3923)
Example 17: [Leu⁸, Asp¹⁰, Ala¹⁶, Gln¹⁸]hPTH (1-36)OH (IS-MS: 4225)
Example 18: [Leu⁸, Asp¹⁰, Lys¹¹, Ala¹⁶, Gln¹⁸, Ala¹⁹, Thr³³]hPTH (1-34)OH (IS-MS: 3999)
Example 19: [Leu⁸, Ala¹⁶, Gln¹⁸, Ala¹⁹]hPTH (1-36)OH (IS-MS: 4166)
Comparative Example 20:[Thr³³]-hPTH-(1-38)-OH

### Comparative EXAMPLE 6: [1-aminocyclopentane-1-carboxylic acid¹-hPTH-(1-36)NH₂

The peptide is assembled in a stepwise manner on a polystyrene based resin support. The Fmoc-group is used for protection of the alpha-amino groups.

Side-chain functional groups are protected as Arg(Pmc), Asn(Trt), Asp(OtBu), Gln(Trt), Glu(OtBu), His(Trt), Lys(Boc), Ser(tBu), Thr(tBu),Trp(Boc) and Tyr(tBu). Other amino acids are left unprotected.

4-(2',4'-Dimethoxyphenyl-Fmoc-amino-methyl)-phenoxy-co(polystyrene-divinylbenzene), 0.4 mmol/g, which may be prepared, e.g., as described in Tetrah. Letters, 28, 3787-3790 (1987) is subjected to the following cycle, steps (1) to (5), of treatments:
(1) DMF
(2) piperidine (20%) in DMF
(3) DMF
(4) mixture of HOBt, diisopropylcarbodiimide, and Fmoc-alanine (0.8 mmol per gram starting resin each)
(5) DMF

Volumes of washes and reagents are from 5 to 20 mL per gram of starting resin.

In the next cycle of treatments (1) to (5), Fmoc-valine is substituted for Fmoc-alanine and so on for each cycle such as to assemble on the resin the correct amino acid sequence of the title compound.

Each step is repeated as many times as necessary for either complete reaction of the resin (steps 2, 4) or complete displacement of the previous reagent(s) from the resin (steps 3, 5). Samples of the resin are removed after each cycle and checked for completeness of the coupling reaction by a colorimetric test for residual amino groups using ninhydrin.

At the end of the synthesis a final cycle comprising steps (1) to (3) only is performed and the peptide resin is washed with 2-propanol, then with a mixture of methanol and methylene chloride (1:1 v/v) and dried throughly in a vacuum desiccator.

The peptide resin (1 g) is suspended in a mixture (20 ml) of trifluoroacetic acid, water, and 1,2-ethanedithiol (90:5:5 v/v) for 2 hours at room temperature, the resin particles are filtered off and washed with a small volume of TFA. The product is precipitated from the combined filtrates by addition of ether (20 volumes), filtered, washed with more ether and dried. The residue is dissolved in 2 % acetic acid, the solution let to stand at r.t. for 8 hours prior to lyophilization. The lyophilisate is chromatographed on a C-18 silica column using a gradient of acetonitrile in 2% H₃P0₄. Fractions are checked by analytical HPLC and those containing the pure compound are collected, filtered through an anion-exchange resin in the acetate form and lyophilised to give the title compound as a polyacetate, polyhydrate.

Fmoc-1-aminocyclopentane-1-carboxylic acid used in the preparation of the peptide resin intermediate may be prepared, e.g. as described by G. Valle et al.,1988, in Can.J.Chem.66:2575-2582. MS(Ion-Spray): 4312

By repeating the procedure as disclosed in Comparative Example 6 but using the appropriate starting materials, following compounds may be obtained:
Comparative Example 21: [Thr³³, Ala³⁴]hPTH (1-34)NH₂
Example 22: [Gln¹⁸]hPTH (1-36)NH₂
Comparative Example 23:[Ala³⁴]-hPTH (1-36)NH₂ (IS-MS: 4210)
Comparative Example 24: [Leu⁸, Asp¹⁰, Lys¹¹, Thr³³, Ala³⁴]hPTH (1-34) NH₂

The compounds of Examples 1 to 24 show correct amino acid ratios in quantitative amino acid analysis.

In the following examples illustrating the recombinant process of the invention, if not otherwise specified, the standard procedures described in Ausubel et al; 1991; Current Protocols in Molecular Biology, John Wiley & Sons, New York are used. The fusion proteins obtained in this process comprising (1-38)PTH are not comprised in the invention.

### Comparative EXAMPLE 25: Cloning bacteriophage T4 gene 55

Two DNA fragments, one containing the entire coding sequence of bacteriophage T4 gene 55, and the other fragment encoding part of it are amplified by polymerase chain reaction (PCR) using purified T4 DNA as the template. Oligonucleotides which contain NdeI or BamHI endonuclease recognition sequences are used as primers. The PCR reactions (25 cycles, each 30" 94°C, 30" 50°C, 30" 72°C) are performed with 1 ng of template and 100 pM of the upstream primer (GAGGTGCATA TGTCAGAAAC TAAGCCT 27), the NdeI recognition site being provided by nucleotides 7-12 thereof) and 100 pM of the downstream primer (GGTCGGATCC ATCGTTAGCG TTAGCCTCAT ATAAAAAATC 40 the BamHI, recognition site being provided by nucleotides 5-10 thereof) in 100 µl reaction buffer containing 10mM Tris-HCl, 50mM KCl, 1.5 mM MgCl₂, 0.2 mM of each of dATP, dCTP, dGTP, dTTP, and 0.1% Triton X 100, pH 9.0. A 0.6kb fragment is obtained. The truncated form of gene 55 is prepared by PCR as described for the entire gene, except that a different oligonucleotide is used as downstream primer (GGTCGGATCC TACGTTGGAC GAATGCAT 28, the BamHI recognition site being provided by nucleotides 5-10 thereof). A 0.35 kb fragment is obtained. The 0.6 kb and 0.35 kb DNA fragments are digested with restriction endonucleases NdeI/BamHI and are cloned into a NdeI/BarnHI digest of expression vector pET17B (obtained from Novagen). The 0.6 kb DNA fragment encodes the entire gp55 protein (188 amino acid residues - see SEQ ID No.1), whereas the 0.35 kb fragment encodes a 12 kD amino-terminal fragment of gp55 (112 amino acid residues - see Fig. 2).

### Comparative Example 26: Preparation and cloning of the DNA encoding PTH (1-38)

DNA fragments encoding a) the linker peptide corresponding to amino acid residues 110-115 of SEQ ID No.1 and PTH (1-38) or b) the linker peptide corresponding to amino acid residues 186-191 of SEQ ID No.3 and PTH (1-38) are generated by PCR using a cloned synthetic PTH (1-38) coding sequence as a template. The oligonucleotides encoding the linker a) (GAGTGATCC ATCGATCCAC CATCCGTATC AGAAATACAA CT 42), or encoding linker b GAGTGGATCC GTTAACGGTC CATCCGTATC AGAAATACAA CT 42 are used as upstream primers. The downstream primer TCTACTCGAG TTAACCCAGA GCTACAAAAT TATG 34

The upstream primers each incorporate a BamHI cloning site and the downstream primer contains a XhoI recognition site (nucleotides 5-10 of each sequence). The PCR reaction is performed under the same conditions as described in Example 25. After cleavage with BamHI and XhoI, the 0.14 kb PCR products are inserted into BamHI-XhoI digests of the gp55-pET17B plasmids, obtained in Example 25. For expression of the fusion proteins the resultant plasmid constructs are transformed into E.coli BL21 (DE3) LysE and single bacterial colonies are further propagated. The expression of the fusion proteins is obtained by incubating a preculture at 37°C on a rotary shaker overnight in circle growth medium (Bio 101). A main culture is inoculated with the preculture making up 1 to 5 % of the volume of the main culture. The main culture is then fermented at a temperature of 37°C and at a pH of 6.9 to 7.1 whilst being aerated at 10 l/min and agitated at 200-400 rpm. Expression is induced by the addition of 1 mM IPTG as soon as the culture reaches an OD₅₅₀ of about 1.0 to 5.0. After induction the fermentation is continued for five hours and then the fermentation broth is harvested without killing the E. coli cells. The harvested cells are spun down with a tubular centrifuge and resuspended in sample buffer and assayed for expression by SDS PAGE. The cell pellet is then frozen until purification. The procedures and host cells used are fully described in Studier et al; 1990; "Use of T7 RNA Polymerase to direct expression of Cloned Genes", Methods in Enzymolog Academic Press. pages 60 to 89.

### Comparative EXAMPLE 27: Preparation of inclusion bodies

Frozen E. coli pellets obtained from Example 26 are resuspended to 25% w/v in Buffer A (50mM Tris PH 8.0; containing 2mM DTT, 5mM Benzamidine-HCl, 1.5mM MgCl₂, 1.0mM MnCl₂, 10pg/ml. DNAse 1 and 2mg/ml Lysozyme) and mixed by stirring for 1 hour at room temperature. The resuspended cells are lysed by passage through a Manton-Gaulin homogeniser (2 passes at 1200 bar) and the resultant lysate kept on ice. The lysate is diluted 2 fold with ice-cold buffer B (50mM Tris pH 8.0; containing 2mM DTT, 5mM Benzamidine-HCl and 4mM EDTA) and centrifuged for 30min at 27,500g. The supernatant from the centrifuge is carefully removed and discarded. The pellet is resuspended in buffer B to 25% w/v, passed once more through the Manton-Gaulin and centrifuged as before. This process is repeated once using buffer B and once using water. The resultant inclusion body pellets are weighed and frozen at -20°C until required.

### Comparative EXAMPLE 28: Solubilisation and cleavage of gp55-Asp-Pro-Pro-(1-38)hPTH inclusion bodies

Frozen inclusion bodies containing gp55-Asp-Pro-Pro-(1-38)hPTH as obtained in Example 27 are suspended in 10 mM HCl acid solution (analytical grade) to a final concentration of 6%w/v. The solution is incubated for 24 hours at 50°C and the reaction terminated by addition of 1 volume of aqueous 100 mM NaOAc solution. The diluted supernatant is clarified by centrifugation at 27,500g for 30 minutes and filtered through a glass fibre filter.

### Comparative EXAMPLE 29: Solubilisation and cleavage of gp55-Asn-Gly-Pro-(1-38)hPTH inclusion bodies

Frozen inclusion bodies containing gp55-Asn-Gly-Pro-(1-38)hPTH and obtained from example 27 are dissolved in 6M Guanidine-HCl (containing 2M Hydroxylamine-HCl and brought to pH 9.0 by addition of 4.5M LiOH) to give an estimated 5mg/ml protein. The pH is readjusted to pH 9 by further addition of LiOH and the solution incubated for 4 hours at 450C. The reaction is terminated by addition of formic acid to pH 4 and the solution centrifuged and filtered as described in example 28.

### Comparative EXAMPLE 30: Preparative HPLC of Gly-Pro and Pro-Pro (1-38)hPTH

Filtered cleavage supernatants are analysed on analytical reversed phase HPLC (Orpogen HD-Gel-RP-7s-300; 4 x 150mm column) and compared with a known (1-38)hPTH standard (Bachem) to determine the PTH concentration. The column is equilibrated with 85% HPLC buffer A (90% water, 10% acetonitrile; containing 0.1%v/v TFA) and 15% HPLC buffer B (10% water, 90% acetonitrile; containing 0.1% TFA) and is eluted with a gradient of 15% HPLC buffer B to 100% HPLC buffer B over 15 minutes at a flow rate of 0.75ml/min. Depending upon the scale of the preparation, supernatants are either loaded on a 1.0 x 25cm C4 Vydac or a 2.2 x 25cm C4 Vydac. The columns are equilibrated with 85% HPLC buffer A and 15% HPLC buffer B at flow rates of 4ml/min and 10ml/min respectively. The columns are eluted with gradients of 15%B to 85%B over 80ml and 15%B to 55%B over 300ml respectively. Gly-Pro-hPTH(1-38) or Pro-Pro-hPTH(1-38) peaks are collected manually and the acetonitrile is later removed under vacuum. Analytical HPLC is used to determine the peptide concentration both after cleavage and in the collected fractions. The solvent free peptide solutions are diluted with an equal volume of 100mM sodium acetate and the pH adjusted, if necessary, to pH 5.4. Following filtration (0.2pm), the solution is loaded on a cation exchange column (either Mono S or SP-Sepharose High Performance, packed into HR10/10 or XK16/10 columns respectively) equilibrated with 50mM sodium acetate pH 5.4 (buffer C). The column is eluted with a 0 to 500mM NaCl gradient in buffer C over 7.5 column volumes. 10ml fractions are collected and the X-X-(1-38)hPTH peak is pooled. The peptide concentration is determined as before using HPLC. This column serves both to exchange the peptides into a more physiological buffer and to remove additional peptides produced by chemical cleavage of the fusion protein itself.

### Comparative EXAMPLE 31: Enzymatic removal of X-X from X-X-(1-38)hPTH

Pooled peptide fractions, as obtained in Example 30, (concentration range 1.5 - 2.0 mg/ml) are brought to pH 8.0 by addition of solid Tris. Purified X-Prolyl dipeptidase (0.5mg/ml in PBS pH 7.2) (Nardi et al. (1991) App. Env. Microbiol. 57, No.1, 45-50) is added to 1:1000 and the solution is incubated for 24h at 37 C. The reaction is stopped by addition of TFA to 0.2% (pH = 5.0) and the resultant (1-38)hPTH is isolated using preparative HPLC (using the same conditions as described in Example 30). The solvent is removed and the product is lyophilised after removal of aliquots for N-terminal sequence and mass spectroscopic analyses.

### Examples of the yields which are obtained using the two fusion protein approaches described above are as follows:

### Gen55-Asp-Pro-Pro-(1-38)PTH (using the "truncated" form of Gen55)

Under partially optimized fermentation conditions, a 215 g wet cell pellet is obtained from a 10 litre fermentation. The percentage level of expression of the fusion protein is approximately 37%. From this pellet, 31 g of inclusion bodies are isolated. The protein purity (with respect to the fusion protein) of these bodies is 61%. After solubilisation, cleavage and centrifugation/filtration approximately 620mg Pro-Pro-PTH are detected. This material yields 580mg Pro-Pro-PTH after subsequent C4-Vydac HPLC. After dilution, cation-exchange on Mono S, digestion with X-Prolydipeptidypeptidase (at a Pro-Pro-PTH concentration of 2mg/ml), HPLC and lyophilisation, 440mg of lyophilized pure product with full biological activity is obtained. This represents a yield of 71% between the stopped cleavage step and the final product.

### Gen55-Asn-Gly-Pro-(1-38)PTH (using the "long" form of Gen55)

From a non-optimized 20 litre fermentation a 78g wet cell pellet with 50% expression of the fusion protein is harvested. Following lysis and centrifugation, 18g of "90% pure" inclusion bodies are obtained. Following the acid stop step and centrifugation/filtration about 470mg Gly-Pro-PTH are detected. After HPLC on C4-Vydac, 355mg of peptide are detected, decreasing to 321mg after SP-Sepharose cation-exchange. Enzymatic removal of the Gly and Pro residues is carried out at 2mg/ml. After the final HPLC step and lyophilization, 244mg of pure, fully biologically active (1-38)PTH is recovered representing a yield of 52% from the stopped cleavage reaction stage.

The compounds of the invention in free form or in the form of pharmaceutically acceptable salts and complexes exhibit valuable pharmacological properties as indicated in animal tests and are therefore indicated for therapy.

The biological activity of the compounds of the invention is assessed in vitro by measuring their ability of stimulating the synthesis of cyclic AMP in UMR 106-06 rat and SaOS-2 human osteosarcoma cells according to the method of Marcus and Aurbach in Endocrinology, 85, 801-810 (1969). Rat osteosarcoma UMR 106 cells are grown to confluence in Eagle's Minimum Essential Medium - 10% FCS in 12 well plates, human SaOS-2 osteosarcoma cells are grown in McCoy's 5A medium - 10% FCS. The medium is then changed to medium with 2% FCS and 1-5 pCi/well [3H]-adenine is added. Two hours later, cells are washed twice with serum-free medium and incubated in DMEM - 1% BSA containing 1 mM 3-isobutyl-1-methylxanthine to exclude actions on phosphodiesterases. Test substances are added 20 min later. The reaction is stopped and cAMP extracted after 15 min by adding ice cold 5% trichloroacetic acid. A carrier solution (0.5 ml/well) containing 5 mM of unlabelled adenine, adenosine, AMP, ADP, ATP, and cAMP as well as 0.4 µCi [14C]-adenosine for determination of recovery is added. [M]-cAMP is separated using serial Dowex 50W-X4 (200-400 mesh) and alumina chromatography and counted. Results are calculated in % of solvent control and EC₅₀ values determined from DRC curves. Compounds of the invention stimulate cAMP production in UMR 106-06 rat and SaOS-2 human osteosarcoma cells at a concentration of 10⁻¹¹ to 10⁻⁶ M.

The compounds of the invention also have binding affinity to PTH receptors, e.g. as follows:

Chicken [Tyr³⁶]PTHrP(1-36)amide is iodinated to a specific activity of 2,200 Ci/mmol using the lactoperoxidase method (Anawa Lab. AG, Wangen). Monolayers of opossum kidney cells are washed with 200 µl DMEM and HAM's F12 (1:1) containing 1% BSA and incubated at 16°C with 50.000cpm of [¹²⁵I-Tyr³⁶]chPTHrP(1-36)amide per well in the presence or absence of 1 µM [Tyr³⁶]chPTHrP(1-36)amide. After incubation, cells are washed with 0.5 ml medium (4°C), lysed with 0.5 ml 1N NaOH and radioactivity is determined. Specific binding is defined as total binding minus nonspecific binding. Competition curves are analyzed using SCTFIT, a non-linear regression computer program (Feyen et al, 1992, Biochem. Biophys. Res. Commun. 187:8-13) and data presented as mean pK_{D} values (n=2 to 3). Compounds of the invention show in this test a binding affinity expressed as a mean pK_{D} value of from 7 to 10.

Furthermore, the compounds of the invention increase plasma calcium level after continuous s.c. infusion, e.g. as determined in male thyroparathyroidectomized Wistar rats weighing 140 to 170 g. 5 days after thyroparathyroidectomy, rats are implanted in the neck with Alzet minipumps and test compounds infused at rates of 0.3 to 30 µg/day/rat. Blood is drawn by retro-orbital puncture in the morning of days 1, 2, 3, and 6 thereafter and plasma calcium concentrations are determined photometrically. In this test compounds of the invention increase plasma calcium.

The compounds of the invention are accordingly indicated for preventing or treating all bone conditions which are associated with increased calcium depletion or resorption or in which calcium fixation in the bone is desirable, e.g. osteoporosis of various genesis (e.g. juvenile, menopausal, post-menopausal, post-traumatic, caused by old age or by cortico-steroid therapy or inactivity), fractures, osteopathy, including acute and chronic states associated with skeletal demineralisation, osteo-malacia, periodontal bone loss or bone loss due to arthritis or osteoarthritis or for treating hypoparathyroidism.

The compounds of the invention are particularly indicated for preventing or treating osteoporosis of various genesis.

For all the above uses, an indicated daily dosage is in the range from about 0.003 to about 10 mg preferably 0.003 to 3, more preferably 0.01 to 1 mg of a compound of the invention. Compounds of the invention may be administered once a day or up to twice a week.

The compounds of the invention may be administered in free form or in pharmaceutically acceptable salt form or complexes. Such salts and complexes may be prepared in conventional manner and exhibit the same order of activity as the free compounds. The present invention also provides a pharmaceutical composition comprising a compound of the invention in free base form or in pharmaceutically acceptable salt form or complex form in association with a pharmaceutically acceptable diluent or carrier. Such compositions may be formulated in conventional manner. The compounds of the invention may be administered by any conventional route, for example parenterally e.g. in form of injectable solutions or suspensions, enterally, e.g. orally, for example in the form of tablets or capsules or in a transdermal, nasal or a suppository form.

In accordance with the foregoing the present invention further provides:
a) a compound of the invention or a pharmaceutically acceptable salt or complex thereof for use as a pharmaceutical;
b) a pharmaceutical composition for improving bone formation, e.g. preventing or treating all bone conditions which are associated with increased calcium depletion or resorption or in which calcium fixation in the bone is desirable, e.g. osteoporosis of various genesis (e.g. juvenile, menopausal, post-menopausal, post-traumatic, caused by old age or by cortico-steroid therapy or inactivity), fractures, osteopathy, including acute and chronic states associated with skeletal demineralisation, osteomalacia, periodontal bone loss or bone loss due to arthritis or osteoarthritis, or for treating hypoparathyroidiom in a subject in need of such treatment; or
c) a compound of the invention or a pharmaceutically acceptable salt or complex thereof for use in the preparation of a pharmaceutical composition for use in the method as in b) above.
   According to a further embodiment of the invention, the compounds of the invention may be employed as adjunct or adjuvant to other therapy, e.g. a therapy using a bone resorption inhibitor, for example as in osteoporosis therapy, in particular a therapy employing calcium, a calcitonin or an analogue or derivative thereof, e.g. salmon, eel or human calcitonin, a steroid hormone, e.g. an estrogen, a partial estrogen agonist or estrogen-gestagen combination, a biphosphonate, vitamin D or an analog thereof, or any combination thereof.
   When the compounds of the invention are administered in conjunction with, e.g. as an adjuvant to bone resorption inhibition therapy, dosages for the co-administered inhibitor will of course vary depending on the type of inhibitor drug employed, e.g. whether it is a steroid or a calcitonin, on the condition to be treated, whether it is a curative or preventive therapy, on the regimen and so forth.
   In accordance with the foregoing the present invention provides in a yet further aspect:
d)a pharmaceutical composition for improving bone formation, e.g. preventing or treating calcium depletion, for example for preventing or treating any of the specific conditions or diseases hereinbefore set forth, in a subject in need of such a treatment comprising a) a compound of the invention and b) a second drug substance, said second drug substance being a bone resorption inhibitor, for example as indicated above.

Compounds of Examples 3,5,8,14 and 15 are preferred.

## Claims

**1.** A Compound of formula I
Ser-Val-Ser-Glu-Ile-Gln-Leu-R₁-His-R₂-R₃-Gly-R₄-His-Leu R₅-R₆-R₇-R₈-Arg-Val-Glu-Trp-Leu-Arg-R₉-Lys-Leu-Gln-Asp-Val-His-R₁₀-R₁₁-(R₁₂)ₙ-X
wherein
R₁ is Met or Leu;
R₂ is Asn, Asp, Gly, Gln, Glu, His, Ser, Thr or Tyr;
R₃ is Leu or Lys;
R₄ is Lys or D- or L-Ala, -Cys, -Gln, -Ile, -Asn, -Trp,-Asp, -Val, -Thr, -Ser, -Tyr, -Met, -Leu or -Gly;
R₅ is Asn, Gln, D-Gln or D- or L-Lys, -Ser. -Leu. -Ala or -Gly;
R₆ is Ser, Asp, Ala. Glu, Gln, Phe, His, Ile or Lys;
R₇ is Gln
R₈ is Glu, Arg, Ala. Val, Tyr, Ser. Lys, Met, His, Gly, Pro, Asn or Ile;
R₉ is Lys, Gln or Arg;
R₁₀ is Asn, Thr, D-Thr or D- or L-Ser, -Leu, -Gly, -Gln, -Arg, -Pro, -Asp, -Ile or -Lys;
R₁₁ is Phe or Ala;
R₁₂ is Val, Val-Ala, Val-Ala-Leu or Val-Ala-Leu-Gly;
n is O or 1; and
X is OH, ORₐ, NH₂ or NR_{b}R_{c} wherein Rₐ is C₁₋₄alkyl, one of
R_{b} and R_{c} is H and the other is C₁₋₆alkyl,
the compound optionally comprising at least one radical selected from a L- or D-a-amino acid, C₂₋₆alkoxy-carbonyl and optionally substituted C₁₋₈alkyl, C₂₋₈alkenyl, C₂₋₈alkynyl, aralkyl, aralkenyl or C₃₋₆cycloalkyl-C₁₋₄alkyl and attached to its terminal amino group, and/or
the compound optionally comprising at least one radical selected from C₂₋₆alkoxycarbonyl and optionally substituted C₁₋₈alkyl, C₂₋₈alkenyl, C₂₋₈alkynyl, aralkyl, aralkenyl or C₃₋₆cycloalkyl-C₁₋₄alkyl attached to one or more side chain amino groups of the compound, in free form or in salt or complex form.

**2.** A compound according to claim 1 wherein R₁₀ is Thr.

**3.** A compound according to claim 1 or 2, wherein R₁₁ is Ala.

**4.** A compound selected from [Leu⁸, Gln¹⁸, Thr³³, Ala³⁴]hPTH, [Leu⁸, Ala¹⁶, Gln¹⁸, Thr³³, Ala³⁴]hPTH, [Leu⁸ Asp¹⁰, Lys¹¹, Ala¹⁶, Gln¹⁸, Thr³³, Ala³⁴]hPTH, [Leu⁸, Asp¹⁰, Lys¹¹, Ala¹⁶, Gln¹⁸]hPTH, [Leu⁸. Ala¹⁶, Gln¹⁸, Ala¹⁹]hPTH, [Leu⁸, Asp¹⁰, Lys¹¹ Gln¹⁸]hPTH [Leu⁸, Asp¹⁰. Lys¹¹, Ala¹⁶, Gln¹⁸, Ala¹⁹]hPTH, [Leu⁸, Ala¹⁶, Gln¹⁸, Ala¹⁹, Thr³³, Ala³⁴]hPTH and [Leu⁸, Asp¹⁰, Lys¹¹, Gln¹⁸, Thr³³, Ala³⁴]hPTH
the compound optionally comprising at least one radical selected from a L- or D-a-amino acid, C₂₋₆alkoxycarbonyl and optionally substituted C₁₋₈alkyl, C₂₋₈alkenyl, C₂₋₈alkynyl, aralkyl, aralkenyl or C₃₋₆cycloalkyl-C₁₋₄alkyl and attached to its terminal amino group,
and/or at least one radical selected from C₂₋₆alcoxycarbonyl and optionally substituted C₁₋₈alkyl, C₂₋₈alkenyl, C₂₋₈ alkynyl, aralkyl. aralkenyl or C₃₋₆cycloalkyl-C₁₋₄alkyl and attached to one or more side chain amino groups of the compound,
in free form or in salt or complex form.
A compound selected from
[Leu⁸,Gln¹⁸, Thr³³, Ala³⁴] 1-hPTH(1-34)OH
[Leu⁸, Ala¹⁶, Gln¹⁸, Ala¹⁹, Thr³³, Ala³⁴]hPTH(1-34)OH
[Leu⁸, Ala¹⁶, Gln¹⁸, Thr³³, Ala³⁴]hPTH(1-34)OH
[Leu⁸, Asp¹⁰, Lys¹¹, Gln¹⁸]hPTH(1-36)OH
[Leu⁸, Asp¹⁰, Lys¹¹,Gln¹⁸, Thr³³, Ala³⁴]hPTH(1-34)OH
[Leu⁸, Asp¹⁰, Lys¹¹, Ala¹⁶, Gln ¹⁸, Ala¹⁹]hPTH(1-36)OH
[Leu⁸, Asp¹⁰, Lys¹¹, Ala¹⁶, Gln¹⁸, Thr³³, Ala³⁴]hPTH(1-34)OH
[Leu⁸, Asp¹⁰, Lys¹¹, Ala¹⁶, Gln¹⁸]hPTH(1-36)OH, and
[Leu⁸, Ala¹⁶,Gln¹⁸, Ala¹⁹]hPTH(1-36)OH
in free form or in salt or complex form.

**6.** A process for the production of a compound according to any one of the preceding claims in which a fusion protein comprising a N-terminal bacteriophage T4 gene 55 polypeptide having the desired compound linked to the C-terminal thereof, is expressed in bacterial cells, and, where required, introducing a C₂₋₆alkoxy-carbonyl or optionally substituted C₁₋₈alkyl, C₂₋₈alkenyl, C₂₋₈alkynyl, aralkyl, aralkenyl or C₃₋₆cycloalkyl-C₁₋₄alkyl on the terminal and/or side chain amino group of the compound in protected or unprotected form and, if required, removing the protecting group present in the protected form.

**7.** A process according to claim 6 in which the fusion protein comprises a chemically cleavable linker between the gene 55 and the desired compound.

**8.** A process according to claim 7 in which the cleavable linker comprises the amino acid sequences Asp-Pro-Pro or Asn-Gly-Pro.

**9**. A nucleotide sequence which codes for a fusion protein comprising an N-terminal bacteriophage T4 gene 55 polypeptide and a desired compound according to any one of the claims 1 to 5, linked to the C-terminal thereof.

**10.** A bacterial expression vector comprising a DNA sequence according to claim 9.

**11.** Bacterial host cells transformed with a DNA sequence according to claim 9, or a bacterial vector according to claim 10.

**12.** A fusion protein comprising an N-terminal bacteriophage T4 gene 55 polypeptide and a desired compound according to any of the claims 1 to 5, linked to the C-terminal thereof.

**13**. A compound according to any one of claims 1 to 5 in free form or in pharmaceutically acceptable salt form for use as a pharmaceutical.

**14.** A pharmaceutical composition comprising a compound as defined in any one of claims 1 to 5 in free form or in pharmaceutically acceptable salt form, together with a pharmaceutically acceptable diluent or carrier therefor.

**15.** A compound according to any one of claims 1 to 5, in free form or in pharmaceutically acceptable salt or complex form, for use in the preparation of a pharmaceutical composition for use in improving bone formation.

**16.** A compound according to any one of claims 1 to 5, in free form or in pharmaceutically acceptable salt or complex form, for use in association with a bone resorption inhibitor to improve bone formation.

## Patentansprüche

1. Verbindung der Formel I
Ser-Val-Ser-Glu-Ile-Gln-Leu-R₁-His-R₂-R₃-Gly-R₄-His-Leu R₅-R₆-R₇-R₈-Arg-Val-Glu-Trp-Leu-Arg-R₉-Lys-Leu-Gln-Asp-Val-His-R₁₀-R₁₁-(R₁₂)ₙ-X
worin
R₁ für Met oder Leu steht,
R₂ für Asn, Asp, Gly, Gln, Glu, His, Ser, Thr oder Tyr steht,
R₃ für Leu oder Lys steht,
R₄ für Lys oder D- oder L-Ala, -Cys, -Gln, -Ile, -Asn, -Trp, -Asp, -Val, -Thr, -Ser, -Tyr, -Met, -Leu oder -Gly steht,
R₅ für Asn, Gln, D-Gln, oder D- oder L-Lys, -Ser, -Leu, -Ala oder -Gly steht,
R₆ für Ser, Asp, Ala, Gln, Gln, Phe, His, Ile oder Lys steht,
R₇ für Gln steht,
R₈ für Glu, Arg, Ala, Val, Tyr, Ser, Lys, Met, His, Gly, Pro, Asn oder Ile steht,
R₉ für Lys, Gln oder Arg steht,
R₁₀ für Asn, Thr, D-Thr oder D- oder L-Ser, -Leu, -Gly, -Gln, -Arg, -Pro, -Asp, -Ile oder Lys steht,
R₁₁ für Phe oder Ala steht,
R₁₂ für Val, Val-Ala, Val-Ala-Leu oder Val-Ala-Leu-Gly steht,
n für O oder 1 steht, und
X für OH, ORₐ, NH₂ oder NR_{b}R_{c} steht, worin Rₐ für C₁-C₄ Alkyl steht, eines von R_{b} und R_{c} für H steht und das andere für C₁-C₆ Alkyl steht,
die Verbindung wahlweise zumindest einen Rest umfaßt, der ausgewählt ist aus einer L- oder D-α-Aminosäure, C₂-C₆ Alkoxycarbonyl und wahlweise substituiertem C₁-C₈ Alkyl, C₂-C₈ Alkenyl, C₂-C₈ Alkinyl, Aralkyl, Aralkenyl oder C₃-C₆ Cycloalkyl-C₁-C₄-alkyl und an die terminale Aminogruppe gebunden ist, und/oder
die Verbindung wahlweise zumindest einen Rest ausgewählt aus C₂-C₆ Alkoxycarbonyl und wahlweise substituiertem C₁-C₈ Alkyl, C₂-C₈ Alkenyl, C₂-C₈ Alkinyl, Aralkyl, Aralkenyl oder C₃-C₆ Cycloalkyl-C₁-C₄-alkyl an eine oder mehrere Seitenkettenaminogruppen der Verbindung gebunden umfaßt, in freier Form oder in Salz- oder Komplexform.

2. Verbindung nach Anspruch 1, worin R₁₀ für Thr steht.

3. Verbindung nach Anspruch 1 oder 2, worin R₁₁ für Ala steht.

4. Verbindung ausgewählt aus
[Leu⁸, Gln¹⁸, Thr³³, Ala³⁴]hPTH
[Leu⁸, Ala¹⁶, Gln¹⁸, Thr³³, Ala³⁴]hPTH
[Leu⁸, Asp¹⁰ Lys¹¹, Ala¹⁶, Gln¹⁸, Thr³³, Ala³⁴]hPTH
[Leu⁸, Asp¹⁰, Lys¹¹, Ala¹⁶, Gln¹⁸]hPTH
[Leu⁸, Ala¹⁶, Gln¹⁸, Ala¹⁹]hPTH
[Leu⁸, Asp¹⁰, Lys¹¹, Gln¹⁸]hPTH
[Leu⁸, Asp¹⁰, Lys¹¹, Ala¹⁶, Gln¹⁸, Ala¹⁹]hPTH
[Leu⁸, Ala¹⁶, Gln¹⁸, Ala¹⁹, Thr³³, Ala³⁴] hPTH und
[Leu⁸, Asp¹⁰, Lys¹¹, Gln¹⁸, Thr³³, Ala³⁴]hPTH
worin die Verbindung wahlweise zumindest einen Rest umfaßt, der ausgewählt ist aus einer L- oder D-α-Aminosäure, C₂-C₆ Alkoxycarbonyl und wahlweise substituiertem C₁-C₈ Alkyl, C₂-C₈ Alkenyl, C₂-C₈ Alkinyl, Aralkyl, Aralkenyl oder C₃-C₆ Cycloalkyl-C₁-C₄-alkyl und an die terminale Aminogruppe gebunden ist, und/oder zumindest einen Rest umfaßt , der ausgewählt ist aus C₂-C₆ Alkoxycarbonyl und wahlweise substituiertem C₁-C₈ Alkyl, C₂-C₈ Alkenyl, C₂-C₈ Alkinyl, Aralkyl, Aralkenyl oder C₃-C₆ Cycloalkyl-C₁-C₄-alkyl und an eine oder mehrere Seitenkettenaminogruppen der Verbindung gebunden ist, in freier Form oder in Salz- oder Komplexform.

5. Verbindung ausgewählt aus
[Leu⁸, Gln¹⁸, Thr³³, Ala³⁴]hPTH(1-34)OH
[Leu⁸, Ala¹⁶, Gln¹⁸, Ala¹⁹, Thr³³, Ala³⁴]hPTH(1-34)OH
[Leu⁸, Ala¹⁶, Gln¹⁸, Thr³³, Ala³⁴]hPTH(1-34)OH
[Leu⁵, Asp¹⁰, Lys¹¹, Gln¹⁸]hPTH(1-36)OH
[Leu⁸, Asp¹⁰, Lys¹¹, Gln¹⁸, Thr³³, Ala³⁴]hPTH(1-34)OH
[Leu⁸, Asp¹⁰, Lys¹¹, Ala¹⁶, Gln¹⁸, Ala¹⁹]hPTH(1-36)OH
[Leu⁸, Asp¹⁰, Lys¹¹, Ala¹⁶, Gln¹⁸, Thr³³, Ala³⁴]hPTH(1-34)OH
[Leu⁸, Asp¹⁰, Lys¹¹, Ala¹⁶, Gln¹⁸]hPTH(1-36)OH und
[Leu⁸, Ala¹⁶, Gln¹⁸, Ala¹⁹]hPTH(1-36)OH
in freier Form oder in Salz- oder Komplexform.

6. Verfahren zur Herstellung einer Verbindung nach einem der vorangehenden Ansprüche, worin ein Fusionsprotein, das ein N-terminales T4 Bakteriophagengen-55-Polypeptid mit der gewünschten Verbindung an den C-Terminus hiervon gebunden umfaßt, in bakteriellen Zellen exprimiert wird, und erforderlichenfalls ein 2-Alkoxycarbonyl oder wahlweise substituiertes C₁-C₈ Alkyl, C₂-C₈ Alkenyl, C₂-C₈ Alkinyl, Aralkyl, Aralkenyl oder C₃-C₆ Cycloalkyl-C₁-C₄-alkyl an die terminale Aminogruppe und/oder eine Seitenkettenaminogruppe der Verbindung in geschützer oder ungeschützter Form eingeführt wird und erforderlichenfalls die in der geschützten Form vorkommende Schutzgruppe entfernt wird.

7. Verfahren nach Anspruch 6, worin das Fusionsprotein einen chemisch spaltbaren Linker zwischen dem Gen 55 und der gewünschten Verbindung enthält.

8. Verfahren nach Anspruch 7, worin der spaltbare Linker die Aminosäuresequenzen Asp-Pro-Pro oder Asn-Gly-Pro umfaßt.

9. Nukleotidsequenz, die für ein Fusionsprotein kodiert, das ein N-terminales T4-Bakteriophagengen-55-Polypeptid und eine gewünschte Verbindung nach einem der Ansprüche 1 bis 5 an den C-Terminus hiervon gebunden umfaßt.

10. Bakterieller Expressionsvektor, der eine DNA Sequenz nach Anspruch 9 umfaßt.

11. Bakerielle Wirtszellen, die mit einer DNA Sequenz nach Anspruch 9 oder einem bakteriellen Vektor nach Anspruch 10 transformiert sind.

12. Fusionsprotein, das ein N-terminales T4-Bakteriophagengen-55-Polypeptid und eine gewünschte Verbindung nach einem der Ansprüche 1 bis 5 an den C-Terminus hiervon gebunden umfaßt.

13. Verbindung nach einem der Ansprüche 1 bis 5 in freier Form oder in pharmazeutisch annehmbarer Salzform zur Verwendung als Pharmazeutikum.

14. Pharmazeutische Zusammensetzung, die eine Verbindung nach einem der Ansprüche 1 bis 5 in freier Form oder in pharmazeutisch annehmbarer Salzform zusammen mit einem pharmazeutisch annehmbaren Verdünnungsmittel oder Träger hierfür enthält.

15. Verbindung nach einem der Ansprüche 1 bis 5 in freier Form oder in pharmazeutisch annehmbarer Salz- oder Komplexform zur Verwendung bei der Herstellung einer pharmazeutischen Zusammensetzung zur Verwendung bei der Verbesserung der Knochenbildung.

16. Verbindung nach einem der Ansprüche 1 bis 5 in freier Form oder in pharmazeutisch annehmbarer Salz- oder Komplexform zur Verwendung zusammen mit einem Knochenresorptionsinhibitor zur Verbesserung der Knochenbildung.

## Revendications

1. Composé de formule I
Ser-Val-Ser-Glu-Ile-Gln-Leu-R₁-His-R₂-R₃-Gly-R₃-His-Leu R₅-R₆-R₇-R₈-Arg-Val-Glu-Trp-Leu-Arg-R₉-Lys-Leu-Gln-Asp∼ Val-His-R₁₀-R₁₁-(R₁₂)ₙ-X
dans laquelle
R₁ représente Met ou Leu ;
R₂ représente Asn, Asp, Gly, Gln, Glu, His, Ser, Thr ou Tyr ;
R₃ représente Leu ou Lys ;
R₄ représente Lys ou D- ou L-Ala, -Cys, -Gln, -Ile, -Asn, -Trp, -Asp, -Val, -Thr, -Ser, -Tyr, -Met, -Leu ou -Gly ;
R₅ représente Asn, Gln, D-Gln ou D- ou L-Lys, -Ser, -Leu, -Ala ou -Gly ;
R₆ représente Ser, Asp, Ala, Glu, Gln, Phe, His, Ile ou Lys ;
R₇ représente Gln ;
R₈ représente Glu, Arg, Ala, Val, Tyr, Ser, Lys, Met, His, Gly, Pro, Asn ou Ile ;
R₉ représente Lys, Gln ou Arg ;
R₁₀ représente Asn, Thr, D-Thr ou D- ou L-Ser, -Leu, -Gly, -Gln, -Arg, -Pro, -Asp, -Ile ou -Lys ;
R₁₁ représente Phe ou Ala ;
R₁₂ représente Val, Val-Ala, Val-Ala-Leu ou Val-Ala-Leu-Gly ;
n vaut 0 ou 1 ; et
X représente OH, ORₐ, NH₂ ou NR_{b}R_{c} où Rₐ représente un alkyle en C₁ à C₄, l'un des radicaux R_{b} et R_{c} représente H, l'autre radical étant un alkyle en C₁ à C₆,
le composé comprenant éventuellement au moins un radical choisi parmi un acide L- ou D-α-aminé, un alcoxy(en C₂ à C₆)-carbonyle et un alkyle en C₁ à C₈, alcényle en C₂ à C₈, alcynyle en C₂ à C₈, aralkyle, aralcényle ou cycloalkyl(en C₃ à C₆)-alkyle en C₁ à C₄ facultativement substitué et attaché à son groupe amino terminal, et/ou
le composé comprenant facultativement au moins un radical choisi parmi un alcoxy(en C₂ à C₆)-carbonyle et un alkyle en C₁ à C₈, alcényle en C₂ à C₈, alcynyle en C₂ à C₈, aralkyle, aralcényle ou cycloalkyl(en C₃ à C₆)-alkyle en C₁ à C₄ éventuellement substitué, attaché à un ou plusieurs groupes amino de chaîne latérale du composé, sous forme libre ou sous forme de sel ou de complexe.

2. Composé selon la revendication 1, dans lequel R₁₀ représente Thr.

3. Composé selon la revendication 1 ou 2, dans lequel R₁₁ représente Ala.

4. Composé choisi parmi [Leu⁸, Gln¹⁸, Thr³³, Ala³⁴]hPTH, [Leu⁸, Ala¹⁶, Gln¹⁸, Thr³³, Ala³⁴]hPTH, [Leu⁸, Asp¹⁰, Lys¹¹, Ala¹⁶, Gln¹⁸, Thr³³, Ala³⁴]hPTH, [Leu⁸, Asp¹⁰, Lys¹¹, Ala¹⁶, Gln¹⁸]hPTH, [Leu⁸, Ala¹⁶, Gln¹⁸, Ala¹⁹]hPTH, [Leu⁸, Asp¹⁰, Lys¹¹, Gln¹⁸]hPTH, [Leu⁸, Asp¹⁰, Lys¹¹, Ala¹⁶, Gln¹⁸, Ala¹⁹]hPTH, [Leu⁸, Ala¹⁶, Glu¹⁸, Ala¹⁹, Thr³³, Ala³⁴]hPTH et [Leu⁸, Asp¹⁰, Lys¹¹, Gln¹⁸, Thr³³, Ala³⁴]hPTH,
le composé comprenant facultativement au moins un radical choisi parmi un acide L- ou D-α-aminé, un alcoxy(en C₂ à C₆)-carbonyle et un alkyle en C₁ à C₈, un alcényle en C₂ à C₈, un alcynyle en C₂ à C₈, un aralkyle, un aralcényle ou un cycloalkyl(en C₃ à C₆)-alkyle en C₁ à C₄ facultativement substitué et attaché à son groupe amino terminal,
et/ou au moins un radical choisi parmi un alcoxy(en C₂ à C₆)-carbonyle et un alkyle en C₁ à C₈, alcényle en C₂ à C₈, alcynyle en C₂ à C₈, aralkyle, aralcényle ou cycloalkyl(en C₃ à C₆)-alkyle en C₁ à C₄ facultativement substitué et attaché à un ou plusieurs groupes amino de chaîne latérale du composé,
sous forme libre ou sous forme de sel ou de complexe.

5. Composé choisi parmi
[Leu⁸, Gln¹⁸, Thr³³, Ala³⁴] 1-hPTH(1-34)OH
[Leu⁸, Ala¹⁶, Gln¹⁸, Ala¹⁹, Thr³³, Ala³⁴] hPTH(1-34)OH
[Leu⁸, Ala¹⁶, Gln¹⁸, Thr³³, Ala³⁴] hPTH(1-34)OH
[Leu⁸, Asp¹⁰, Lys¹¹, Gln¹⁸] hPTH(1-36)OH
[Leu⁸, Asp¹⁰, Lys¹¹, Gln¹⁸, Thr³³, Ala³⁴] hPTH(1-34)OH
[Leu⁸, Asp¹⁰, Lys¹¹, Ala¹⁶, Gln¹⁸, Ala¹⁹] hPTH(1-36)OH
[Leu⁸, Asp¹⁰, Lys¹¹, Ala¹⁶, Gln¹⁸, Thr³³, Ala³⁴] hPTH(1-34)OH
[Leu⁸, Asp¹⁰, Lys¹¹, Ala¹⁶, Gln¹⁸] hPTH(1-36)OH, et
[Leu⁸, Ala¹⁶, Gln¹⁸, Ala¹⁹] hPTH(1-36)OH
sous forme libre ou sous forme de sel ou de complexe.

6. Procédé de production d'un composé selon l'une quelconque des revendications précédentes dans lequel une protéine de fusion comprenant un polypeptide du gène 55 de bactériophage T4 N-terminal ayant le composé désiré lié à son extrémité C-terminale est exprimé dans des cellules bactériennes et, lorsque cela est nécessaire, on introduit un alcoxy-(en C₂ à C₆)-carbonyle ou un alkyle en C₁ à C₈, un alcényle en C₂ à C₈, un alcynyle en C₂ à C₈, un aralkyle, un aralcényle ou un cycloalkyl(en C₃ à C₆)-alkyle en C₁ à C₄ facultativement substitué, sur le groupe amino terminal et/ou de chaîne latérale du composé sous forme protégée ou non protégée et, si on le désire, on retire le groupe protecteur présent sous forme protégée.

7. Procédé selon la revendication 6 dans lequel la protéine de fusion comprend un lieur chimiquement clivable entre le gène 55 et le composé désiré.

8. Procédé selon la revendication 7 dans lequel le lieur clivable comprend les séquences d'acides aminés Asp-Pro-Pro ou Asn-Gly-Pro.

9. Séquence de nucléotides qui code pour une protéine de fusion comprenant un polypeptide du gène 55 de bactériophage T4 N-terminal et un composé désiré selon l'une quelconque des revendications 1 à 5, lié à son extrémité C-terminale.

10. Vecteur d'expression bactérienne comprenant une séquence d'ADN selon la revendication 9.

11. Cellules hôtes bactériennes transformées au moyen d'une séquence d'ADN selon la revendication 9, ou d'un vecteur bactérien selon la revendication 10.

12. Protéine de fusion comprenant un polypeptide du gène 55 de bactériophage T4 N-terminal et un composé désiré selon l'une quelconque des revendications 1 à 5, lié à son extrémité C-terminale.

13. Composé selon l'une quelconque des revendications 1 à 5 sous forme libre ou sous forme de sel pharmaceutiquement acceptable aux fins d'utilisation comme produit pharmaceutique.

14. Composition pharmaceutique comprenant un composé tel que défini dans l'une quelconque des revendications 1 à 5 sous forme libre ou sous forme de sel pharmaceutiquement acceptable, avec un diluant ou véhicule pharmaceutiquement acceptable approprié.

15. Composé selon l'une quelconque des revendications 1 à 5, sous forme libre ou sous forme de sel ou de complexe pharmaceutiquement acceptable, aux fins d'utilisation dans la préparation d'une composition pharmaceutique destinée à être utilisée pour améliorer la formation des os.

16. Composé selon l'une quelconque des revendications 1 à 5, sous forme libre ou sous forme de sel ou de complexe pharmaceutiquement acceptable, aux fins d'utilisation en association avec un inhibiteur de résorption osseuse pour améliorer la formation de l'os.
